# EUROPEAN PATENT SPECIFICATION

(11) **EP 4 037 561 B1**
(45) Date of publication and mention of the grant of the patent: **18.06.2025**
(21) Application number: 20789287.8
(22) Date of filing: 18.09.2020
(51) Int. Cl.: A61B 5/053

(54) **BODY ELECTRODE UNIT**
KÖRPERELEKTRODENEINHEIT
UNITÉ D'ÉLECTRODE CORPORELLE

(30) Priority: 04.10.2019 JP 2019183803
(43) Date of publication of application: 10.08.2022
(73) Proprietor: Nihon Kohden Corporation, Shinjuku-ku Tokyo 161-8560 (JP)
(72) Inventor: ODAKA, Ryugo, Tokorozawa-shi, Saitama 359-0037 (JP); NISHIWAKI, Shigehiro, Tokorozawa-shi, Saitama 359-0037 (JP)
(74) Representative: Grünecker Patent- und Rechtsanwälte PartG mbB
(86) International application number: PCT/JP2020/035545
(87) International publication number: WO 2021/065580

(56) References cited:
- WO-A1-02/22010
- US-A- 4 635 641
- US-A1- 2010 210 965
- US-A1- 2016 166 170
- US-A1- 2017 258 402

## Description

### Technical Field

The presently disclosed subject matter relates to a body electrode unit configured to be used in measurement of physiological information.

### Background

A related art body electrode is provided in a state where a face on which an adhesive gel is disposed is attached to a release sheet. When using the body electrode, the body electrode is peeled off from the release sheet, the face on which the adhesive gel is disposed is attached to a body.

The body electrode may have a plurality of electrodes mounted on a substrate sheet that is separable into a plurality of areas (see, e.g., JP2007-050033A). In such a body electrode, the electrodes are put together to form a single continuous shape, and the substrate sheet is attached to a plurality of liners. When the plurality of liners are peeled off, therefore, the electrodes that are put together to form a single continuous shape can be attached to a body at once. As compared with an example where electrodes are attached one by one, the attaching can be easily performed.

When attaching the plurality of electrodes of the related art body electrode to a body at once, however, the substrate sheet can be attached to an unintended position, which may cause a failure of the body electrode.

US 2017/258402 A1 relates to a wireless sensor that offers convenient use for a subject in the monitoring, in real time of a medical signal.

US 4 635 641 A relates to a multi-element body electrode for therapeutic and diagnostic applications in medical and veterinary use.

### Summary

Illustrative aspects of the presently disclosed subject matter provide a body electrode unit having a plurality of electrodes that are put together to form a single continuous shape, with which each electrode can be attached to a desired position on a body.

According to an aspect of the presently disclosed subject matter, a body electrode unit includes a body electrode configured to be attached to a body, and a release sheet to which the body electrode is attached. The body electrode includes at least a first electrode and a second electrode. The first electrode and the second electrode are put together to form a single continuous shape. The release sheet includes a first processed part configured to facilitate at least one of a separation and a bending of the release sheet, the first processed part being provided in a first area between a position at which the first electrode is attached and a position at which the second electrode is attached.

### Brief Description of Drawings

[fig.1A] FIG. 1A is a view illustrating a front side a body electrode.
[fig.1B]FIG. 1B is a view illustrating a back side a body electrode.
[fig.2A]FIG. 2A is a view illustrating a body electrode unit.
[fig.2B]FIG. 2B is another view illustrating a body electrode unit.
[fig.3]FIG. 3 is a view illustrating a lateral side of the body electrode unit.
[fig.4A]FIG. 4A is a view illustrating how the body electrode unit is used.
[fig.4B]FIG. 4B is another view illustrating how the body electrode unit is used.
[fig.4C]FIG. 4C is another view illustrating how the body electrode unit is used.
[fig.4D]FIG. 4D is another view illustrating how the body electrode unit is used.
[fig.5A]FIG. 5A is another view illustrating how the body electrode unit is used.
[fig.5B]FIG. 5B is another view illustrating how the body electrode unit is used.
[fig.5C]FIG. 5C is another view illustrating how the body electrode unit is used.
[fig.5D]FIG. 5D is another view illustrating how the body electrode unit is used.
[fig.6A]FIG. 6A is a view illustrating an example of first and second connection portions.
[fig.6B]FIG. 6B is a view illustrating another example of first and second connection portions.
[fig.6C]FIG. 6C is a view illustrating another example of first and second connection portions.
[fig.7A]FIG. 7A is a view illustrating an example a neutral electrode.
[fig.7B]FIG. 7B is another view illustrating the example the neutral electrode.

### Description of Embodiments

Hereinafter, embodiments of the presently disclosed subject matter will be described with reference to the drawings.

### Structure of Body Electrode 1

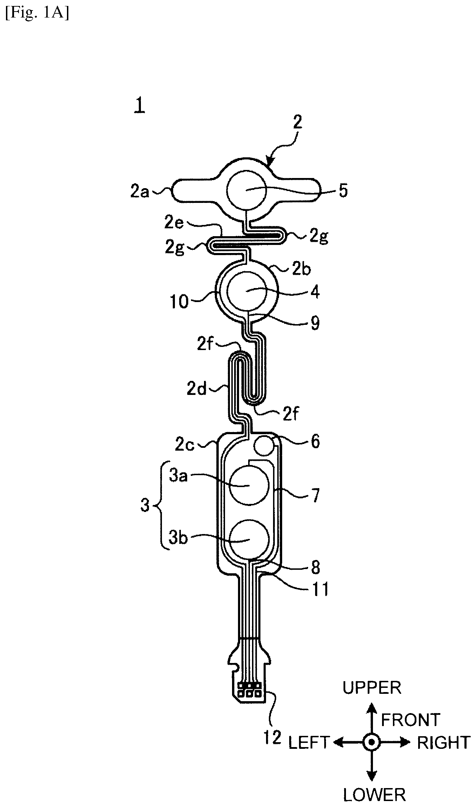

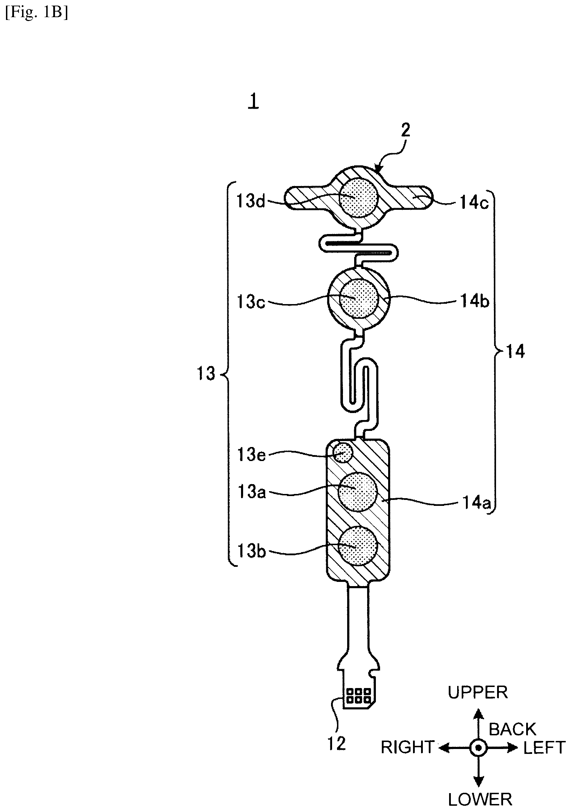

First, a structure of a body electrode 1 will be described with reference to FIGS. 1A and 1B. FIG. 1A is a front view of the body electrode 1, and FIG. 1B is a back view of the body electrode 1.

In this example, the body electrode 1 is used to stimulate a nerve leading to a muscle of a body (a subject to which the body electrode 1 is attached), and to monitor the degree of relaxation of the reacting muscle based on a physiological signal of the muscle. For example, the body electrode 1 is used in the case where the degree of relaxation of a muscle of a body is monitored by the TOF (Train of Four) method in which muscle stimulation is successively performed four times every 0.5 seconds, and the process of four successive muscle stimulations is repeated every 15 seconds.

A stimulation electrode 3, an active electrode 4, a reference electrode 5, and a neutral electrode 6 are mounted on a base 2. The base 2 includes an upper island portion 2a on which the reference electrode 5 is mounted, an intermediate island portion 2b on which the active electrode 4 is mounted, a lower island portion 2c on which the stimulation electrode 3 and the neutral electrode 6 are mounted, a first connection portion 2d provided between the intermediate island portion 2b and the lower island portion 2c, and a second connection portion 2e provided between the upper island portion 2a and the intermediate island portion 2b.

The first connection portion 2d has a first direction changing part 2f configured to change the direction in which the first connection portion 2d extends. Since the first connection portion 2d has the first direction changing part 2f, at least one of the distance and the angle between the stimulation electrode 3 and the active electrode 4 is adjustable.

The second connection portion 2e has a second direction changing part 2g configured to change the direction in which the second connection portion 2e extends. Since the second connection portion 2e has the second direction changing part 2g, at least one of the distance and the angle between the active electrode 4 and the reference electrode 5 is adjustable.

The first direction changing part 2f is configured to change the direction in which the first connection portion 2d extends in the longitudinal direction of the body electrode 1. The second direction changing part 2g is configured to change the direction in which the second connection portion 2e extends in the lateral direction of the body electrode 1. That is, the first direction changing part 2f and the second direction changing part 2g are configured to change the direction in which the first connection portion 2d extends and the direction in which the second connection portion 2e extends in different directions, respectively.

The first direction changing part 2f is configured to change the direction in which the first connection portion 2d extends in the longitudinal direction of the body electrode 1 to prevent, when attaching the body electrode 1 to the subject, the first connection portion 2d from rising up and from contacting the wrist of the subject to stimulate the sensation of pain.

The second direction changing part 2g is configured to change the direction in which the second connection portion 2e extends in the lateral direction of the body electrode 1 to prevent, when attaching the body electrode 1 the subject, the second connection portion 2e from rising up and from contacting the hypothenar to stimulate the sensation of pain.

The stimulation electrode 3 is disposed at a position closer to a connector 12 in the longitudinal direction of the body electrode 1 as compared with the reference electrode 5, and is configured to apply electrical stimulation to a nerve leading to a muscle of the body (for example, the ulnar nerve). The stimulation electrode 3 includes a negative electrode 3a and a positive electrode 3b.

The active electrode 4 is disposed between the stimulation electrode 3 and the reference electrode 5, and provided to detect a physiological signal from a muscle that responds to the electrical stimulation applied by the stimulation electrode 3. In the embodiment, the active electrode 4 is configured by a cathode. Here, the active electrode 4 is mounted corresponding to, for example, the abductor digiti minimi muscle.

The reference electrode 5 is disposed at a position farther from the connector 12 in the longitudinal direction of the body electrode 2 with respect to the stimulation electrode 3 and the active electrode 4, and is configured to detect a physiological signal from a muscle that responds to the electrical stimulation applied by the stimulation electrode 3. In the embodiment, the reference electrode 5 is a positive electrode. Here, the reference electrode 5 is mounted corresponding to, for example, the abductor digiti minimi muscle.

The neutral electrode 6 is provided to block noises flowing through the body electrode 1. The neutral electrode 6 is disposed proximal to the negative electrode 3a. Therefore, noises that are generated in the case where the negative electrode 3a and the active electrode 4 are close to each other are easily blocked.

A negative electrode wiring part 7 is provided to connect the negative electrode 3a and the connector 12 to each other. Based on an operation performed on an electric stimulator (not illustrated), therefore, the negative electrode 3a applies stimulation to the body through the negative electrode wiring part 7.

A positive electrode wiring part 8 is provided to connect the positive electrode 3b and the connector 12 to each other. Based on an operation performed on the electric stimulator, therefore, the positive electrode 3b applies stimulation to the body through the positive electrode wiring part 8.

An active electrode wiring part 9 is provided to connect the active electrode 4 and the connector 12 to each other. Based on a detection of a physiological signal of the living bode, therefore, the active electrode 4 outputs the physiological signal to the electric stimulator through the active electrode wiring part 9. A part of the active electrode wiring part 9 is arranged in the first connection portion 2d.

A reference electrode wiring part 10 is provided to connect the reference electrode 5 and the connector 12 to each other. Based on a detection of a physiological signal of the living bode, therefore, the reference electrode 5 outputs the physiological signal to the electric stimulator through the reference electrode wiring part 10. A part of the reference electrode wiring part 10 is arranged in the second connection portion 2e.

A neutral electrode wiring part 11 is provided to connect the neutral electrode 6 and the connector 12 to each other.

The connector 12 is provided to connect the wiring parts respectively connected to the electrodes. Specifically, the negative electrode wiring part 7 connected to the negative electrode 3a, the positive electrode wiring part 8 connected to the positive electrode 3b, the active electrode wiring part 9 connected to the active electrode 4, the reference electrode wiring part 10 connected to the reference electrode 5, and the neutral electrode wiring part 11 connected to the neutral electrode 6 are connected to the connector 12. The connector 12 is further connected to the electric stimulator. Spare connecting parts that are not connected to any wiring part are disposed in the connector 12.

An adhesive gel 13 is provided to attach the electrodes to the body, and covers the back sides of the electrodes. Specifically, the adhesive gel 13 includes a negative electrode adhesive gel 13a that covers the back side of the negative electrode 3a, a positive electrode adhesive gel 13b that covers the back side of the positive electrode 3b, an active electrode adhesive gel 13c that covers the back side of the active electrode 4, a reference electrode adhesive gel 13d that covers the back side of the reference electrode 5; and a neutral electrode adhesive gel 13e that covers the back side of the neutral electrode 6.

An adhesive tape 14 is provided to attach the body electrode 1 to a release sheet 100 that will be described later, and has an adhesiveness. As illustrated in FIG. 1B, the adhesive tape 14 has a configuration including: a stimulation electrode adhesive tape 14a that is disposed on the back side of the lower island portion 2c, and in the peripheries of the negative electrode adhesive gel 13a, the positive electrode 3b, and the neutral electrode adhesive gel 13e; an active electrode adhesive tape 14b that is disposed on the back side of the intermediate island portion 2b, and in the periphery of the active electrode adhesive gel 13c; and a reference electrode adhesive tape 14c that is disposed on the back side of the upper island portion 2a, and in the periphery of the reference electrode adhesive gel 13d.

In place of the adhesive tape 14, for example, a configuration in which paste is attached to the upper island portion 2a, the intermediate island portion 2b, and the lower island portion 2c, and which is attached to the release sheet 100 may be employed. That is, it is requested only to perform a process so as to provide the upper island portion 2a, the intermediate island portion 2b, and the lower island portion 2c with an adhesiveness. Here, "adhesiveness" means possession of a viscosity of a degree at which the portions are stuck and held to the release sheet 100 or the skin of the subject.

An adhesive tape is not disposed on the back side of the first connection portion 2d, and that of the second connection portion 2e. That is, the back side of the first connection portion 2d, and that of the second connection portion 2e do not have an adhesiveness. When the stimulation electrode adhesive tape 14a, the active electrode adhesive tape 14b, and the reference electrode adhesive tape 14c are to be attached to the subject, therefore, a situation where the tapes are attached to unintended positions can be prevented from occurring.

### Body Electrode Unit U

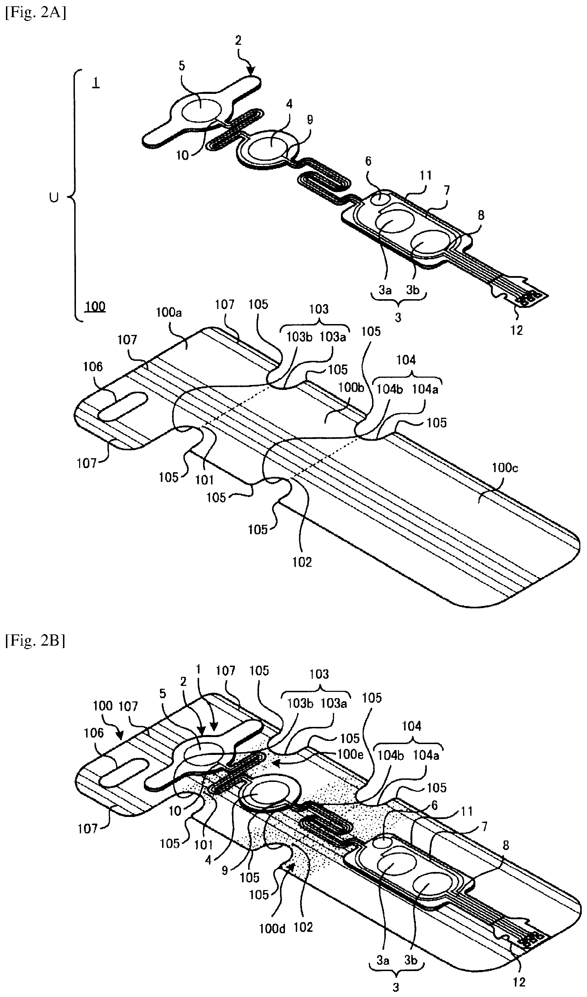

Referring to FIGS. 2A and 2B, next, a body electrode unit U will be described. FIG. 2A is an exploded perspective view of the body electrode unit U, and FIG. 2B is a perspective view of the body electrode unit U.

As illustrated in FIG. 2A, the body electrode unit U has the body electrode 1 that has been described with reference to FIGS. 1A and 1B, and the release sheet 100.

The release sheet 100 is a sheet to which the body electrode 1 is to be attached, and can transmit light. During a process for producing the body electrode unit U, therefore, it is easily check whether the adhesive gel 13 is provided cover the back sides of the electrodes.

The release sheet 100 has: an upper sheet portion 100a to which the reference electrode adhesive tape 14c of the upper island portion 2a is to be attached; an intermediate sheet portion 100b to which the active electrode adhesive tape 14b of the intermediate island portion 2b is to be attached; and a lower sheet portion 100c to which the stimulation electrode adhesive tape 14a of the lower island portion 2c is to be attached. The release sheet 100 includes a first processed part 101, a second processed part 102, a first notch 103, a second notch 104, rounded parts 105, a positioning hole 106, and patterned portions 107.

A process for facilitating separation and/or bending of the upper sheet portion 100a and the intermediate sheet portion 100b is applied to the first processed part 101.

A process for facilitating separation and/or bending of the intermediate sheet portion 100b and the lower sheet portion 100c is applied to the second processed part 102.

In the embodiment, a perforation process is performed in the first processed part 101 and the second processed part 102 as a mode in which the release sheet 100 can be separated and/or bent. However, the mode is not limited to this, and any mode may be used in so far as it can separate and/or bend the release sheet 100. For example, a half-cut process in which a part of the thickness of the release sheet 100 is cut may be applied to the first processed part 101 and the second processed part 102. In the thickness (e.g., about 75 µm) of the release sheet 100, specifically, the cutting is performed at a predetermined thickness (e.g., about 50 µm) that is smaller than the thickness of the release sheet 100, thereby enabling the release sheet 100 to be separated and/or bent.

In the embodiment, the first processed part 101 may be disposed at any position in so far as it is in a first area 100d that, in the case where the body electrode 1 is attached to the release sheet 100, is between the lower end of the intermediate island portion 2b and the upper end of the lower island portion 2c. In the same or similar manner, the second processed part 102 may be disposed at any position in so far as it is in a second area 100e that, in the case where the body electrode 1 is attached to the release sheet 100, is between the lower end of the upper island portion 2a and the upper end of the intermediate island portion 2b.

The first notch 103 is disposed in the both side ends of the first processed part 101. In the embodiment, as illustrated in FIGS. 2A and 2B, the first notch 103 has a configuration including a first right notch 103a that is disposed in the right side end of the first processed part 101, and a first left notch 103b that is disposed in the left side end of the first processed part 101. Each of the notches has an approximately semicircular shape.

The second notch 104 is disposed in the both side ends of the second processed part 102. In the embodiment, as illustrated in FIGS. 2A and 2B, the second notch 104 has a configuration including a second right notch 104a that is disposed in the right side end of the second processed part 102, and a second left notch 104b that is disposed in the left side end of the second processed part 102. Each of the notches has an approximately semicircular shape.

Since the first notch 103 is disposed, the width of the first processed part 101 is smaller than that of the release sheet 100. In the same or similar manner, since the second notch 104 is disposed, the width of the second processed part 102 is smaller than that of the release sheet 100. Because of these, a situation can be prevented from occurring where, when the upper sheet portion 100a and the intermediate sheet portion 100b are to be separated from each other along the first processed part 101, or when the intermediate sheet portion 100b and the lower sheet portion 100c are to be separated from each other along the second processed part 102, the first connection portion 2d or the second connection portion 2e is stretched without slack during the course of the separation, and the release sheet 100 cannot be cut off up to the end.

The shape of the first notch 103, and that of the second notch 104 are not limited to an approximately semicircular shape, and may be any shape in so far as it can make the width of the first processed part 101 smaller than that of the release sheet 100, and that of the second processed part 102 smaller than that of the release sheet 100.

The rounded parts 105 are disposed in end parts of the first notch 103 and the second notch 104. Since the rounded parts 105 are disposed, here, a situation can be prevented from occurring where, when the release sheet 100 is to be cut off along the first processed part 101, or when the release sheet 100 is to be cut off along the second processed part 102, sharp edges are formed, and someone is injured.

The positioning hole 106 is provided to fix the release sheet 100 during a process for producing the body electrode unit U.

The patterned portions 107 are provided linearly in the longitudinal direction of the release sheet 100. Since the patterned portions 107 are disposed, the release sheet 100 can be easily recognized when it is transparent and is dropped on the floor. Therefore, it is possible to prevent someone from slipping by stepping on the release sheet 100. The patterned portions 107 may have any pattern in so far as the pattern enables the release sheet 100 to be easily recognized.

### Lateral Side of Body electrode Unit U

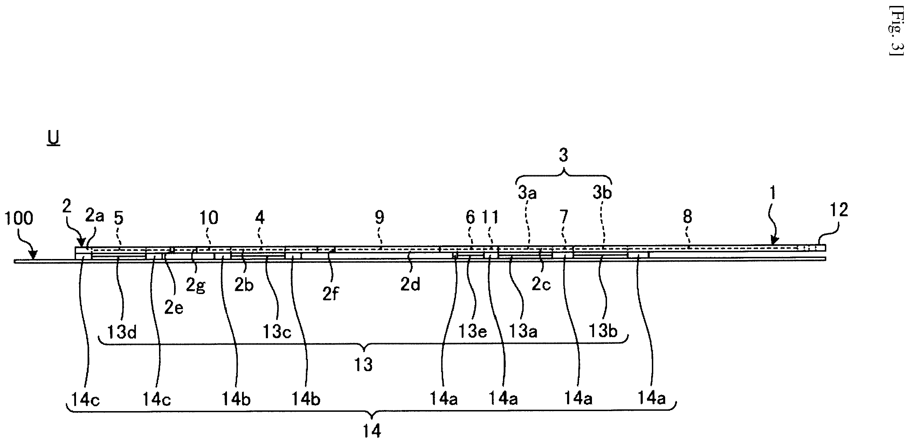

Referring to Fig. 3, next, a side view of the body electrode unit U will be described.

As illustrated in FIG. 3, the body electrode 1 is configured such that the stimulation electrode 3, the active electrode 4, and the reference electrode 5 are put together to form a single continuous shape. Specifically, the intermediate island portion 2b where the active electrode 4 is mounted, and the lower island portion 2c where the stimulation electrode 3 is mounted are connected to each other by the first connection portion 2d. The upper island portion 2a where the reference electrode 5 is mounted, and the intermediate island portion 2b where the active electrode 4 is mounted are connected to each other by the second connection portion 2e. This is how the stimulation electrode 3, the active electrode 4, and the reference electrode 5 are put together to form a single continuous shape in this example.

### Use of Body Electrode Unit U

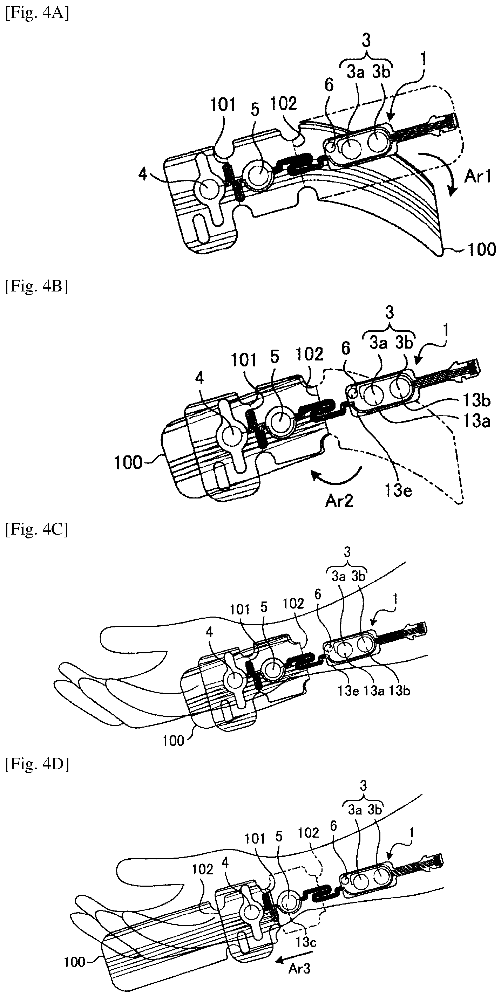

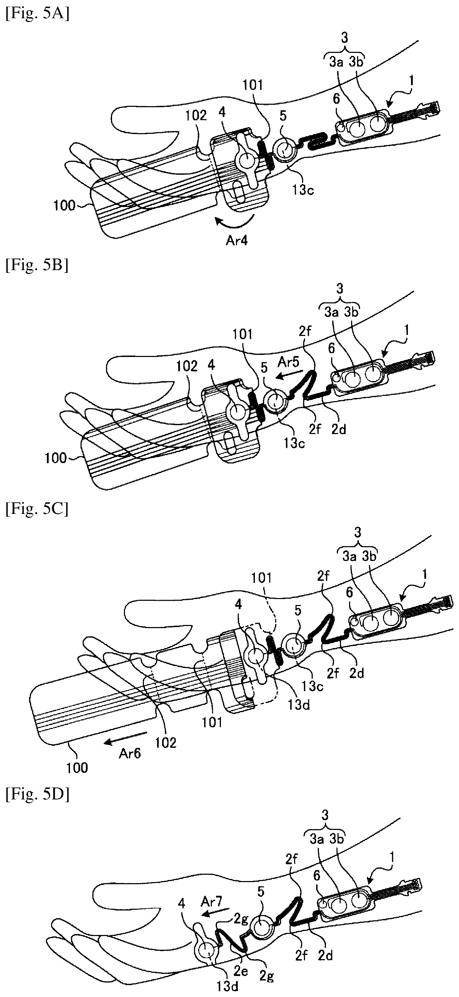

Referring to FIGS. 4 and 5, next, a use mode of the body electrode unit U will be described.

As described above, the body electrode 1 is attached to the release sheet 100. In the case where the body electrode 1 is to be used, therefore, the release sheet 100 is first moved in the direction of the arrow Ar1 as illustrated in FIG. 4A, whereby the attaching between the lower sheet portion 100c and the stimulation electrode adhesive tape 14a is released. As a result, the release sheet 100 is peeled off in a range extending to the second processed part 102.

As illustrated in FIG. 4B, next, the release sheet 100 is moved in the direction of the arrow Ar2, thereby bending the release sheet 100 along the second processed part 102. In the case where the second processed part 102 is configured by tearable perforations, although not illustrated, the lower sheet portion 100c may be separated from the release sheet 100 along the second processed part 102.

As illustrated in FIG. 4C, next, the stimulation electrode adhesive tape 14a is attached so that the negative electrode adhesive gel 13a and the positive electrode adhesive gel 13b correspond to the ulnar nerve of the subject.

As illustrated in FIG. 4D, next, the release sheet 100 is moved in the direction of the arrow Ar3, whereby the attaching between the intermediate sheet portion 100b and the active electrode adhesive tape 14b is released. As a result, the release sheet 100 is peeled off in a range extending to the first processed part 101.

As illustrated in FIG. 5A, next, the release sheet 100 is moved in the direction of the arrow Ar4, thereby bending the release sheet 100 along the first processed part 101. In the case where the first processed part 101 is configured by tearable perforations, although not illustrated, the intermediate sheet portion 100b may be separated from the upper sheet portion 100a along the first processed part 101.

As illustrated in FIG. 5B, next, the active electrode adhesive tape 14b is attached so that the active electrode adhesive gel 13c corresponds to the abductor digiti minimi muscle of the subject.

At this time, the first direction changing part 2f that can change at least one of the distance and angle between the stimulation electrode 3 and the active electrode 4 is disposed in the first connection portion 2d, and therefore the first connection portion 2d elongates in the direction of the arrow Ar5.

As illustrated in FIG. 5C, next, the release sheet 100 is moved in the direction of the arrow Ar6, whereby the attaching between the upper sheet portion 100a and the reference electrode adhesive tape 14c is released. As a result, the release sheet 100 is peeled off from the body electrode 1.

As illustrated in FIG. 5D, next, the reference electrode adhesive tape 14c is attached so that the reference electrode adhesive gel 13d corresponds to the abductor digiti minimi muscle of the subject.

At this time, the second direction changing part 2g that can change at least the distance and angle between the active electrode 4 and the reference electrode 5 is disposed in the second connection portion 2e, and therefore the second connection portion 2e elongates in the direction of the arrow Ar7.

As described above, the first direction changing part 2f is disposed in the first connection portion 2d in the embodiment, and therefore the first connection portion 2d can elongate. Moreover, the second direction changing part 2g is disposed in the second connection portion 2e in the embodiment, and therefore the second connection portion 2e can elongate. Because of these, in the body electrode 1 in which the stimulation electrode 3, the active electrode 4, and the reference electrode 5 are put together to form a single continuous shape, even in the case where the subject has a large hand, the lower island portion 2c where the stimulation electrode 3 is mounted, the intermediate island portion 2b where the active electrode 4 is mounted, and the upper island portion 2a where the reference electrode 5 is mounted can be attached to desired positions respectively.

Since the first connection portion 2d can elongate, and the second connection portion 2e can elongate, the body electrode 1 of the embodiment can be used not only while the lower island portion 2c where the stimulation electrode 3 is mounted, the intermediate island portion 2b where the active electrode 4 is mounted, and the upper island portion 2a where the reference electrode 5 is mounted are attached to the hand or arm of the subject, but also while these portions are attached to the foot of the subject.

On the other hand, also in the case where the body electrode 1 in which the stimulation electrode 3, the active electrode 4, and the reference electrode 5 are put together to form a single continuous shape is used in a subject having a small hand, there is a problem in that the stimulation electrode 3, the active electrode 4, and the reference electrode 5 are hardly attached to desired positions.

In the embodiment, by contrast, the first direction changing part 2f is disposed in the first connection portion 2d, and therefore the first connection portion 2d can be shortened. In the embodiment, furthermore, the second direction changing part 2g is disposed in the second connection portion 2e, and therefore the second connection portion 2e can be shortened. Because of these, in the body electrode 1 in which the stimulation electrode 3, the active electrode 4, and the reference electrode 5 put together to form a single continuous shape, even in the case where the subject has a small hand, the lower island portion 2c where the stimulation electrode 3 is mounted, the intermediate island portion 2b where the active electrode 4 is mounted, and the upper island portion 2a where the reference electrode 5 is mounted can be attached to desired positions.

In the case where the subject has a small hand, when the first connection portion 2d is shortened by the first direction changing part 2f, the negative electrode 3a configured by a cathode, and the active electrode 4 configured by a cathode are close to each other. Therefore, it may be considered that a detected physiological signal is made unstable by noises. However, the neutral electrode 6 is disposed proximal to the negative electrode 3a. Even in the case where the negative electrode 3a and the active electrode 4 are close to each other, therefore, noises can be efficiently blocked, and hence a detected physiological signal can be stabilized.

### Examples of First Connection Portion 2d and Second Connection Portion 2e

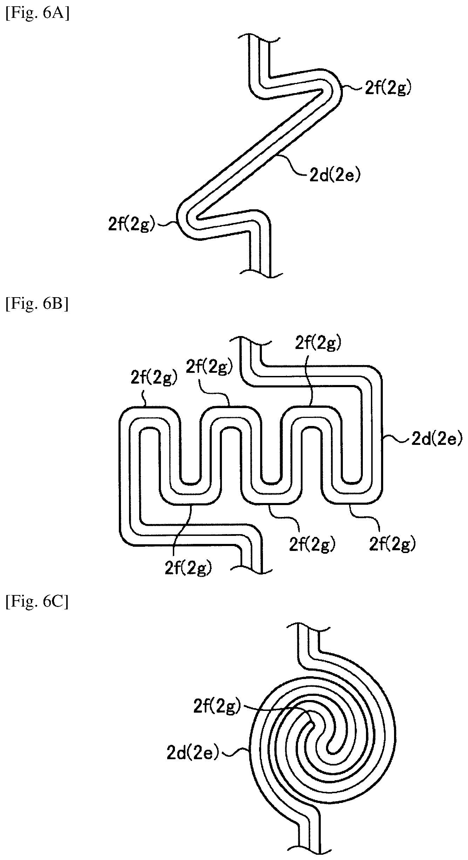

Referring to FIGS. 6A to 6C, other examples of the first connection portion 2d and the second connection portion 2e will be described.

FIG. 6A illustrates an example of the first connection portion 2d and the second connection portion 2e. In this example, in the first connection portion 2d and the second connection portion 2e, each of the first direction changing part 2f and the second direction changing part 2g is disposed at two locations, and are configured to change the directions in which the first connection portion 2d and the second connection portion 2e extend in oblique directions.

FIG. 6B illustrates another example of the first connection portion 2d and the second connection portion 2e. In this example, in the first connection portion 2d and the second connection portion 2e, each of the first direction changing part 2f and the second direction changing part 2g is disposed at six locations, and are configured to change the first connection portion 2d and the second connection portion 2e in the longitudinal direction of the body electrode.

FIG. 6C illustrates another example of the first connection portion 2d and the second connection portion 2e. In this example, in the first connection portion 2d and the second connection portion 2e, each of the first direction changing part 2f and the second direction changing part 2g is disposed at one location, and are configured to change the direction in which the first connection portion 2d and the second connection portion 2ec extend in a rotational direction.

As described above, the direction in which the first connection portion 2d is changed by the first direction changing part 2f, and the direction in which the second connection portion 2e is changed by the second direction changing part 2g can be adequately set.

The number of the first direction changing part 2f that is provided in the first connection portion 2d is required to be at least one, and may be adequately set.

In the same or similar manner, the number of the second direction changing part 2g that is provided in the second connection portion 2e is required to be at least one, and may be adequately set.

### Another Example of Neutral Electrode 6

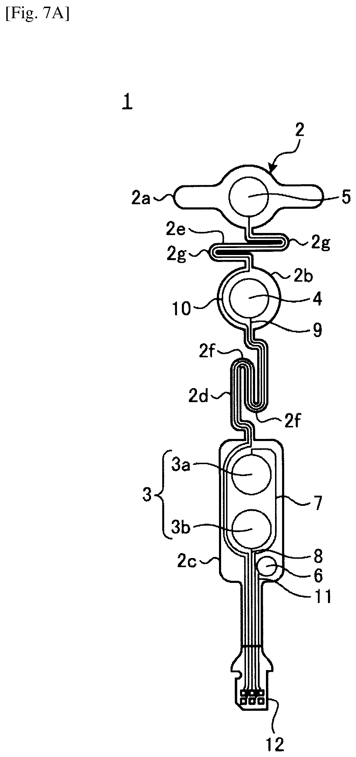

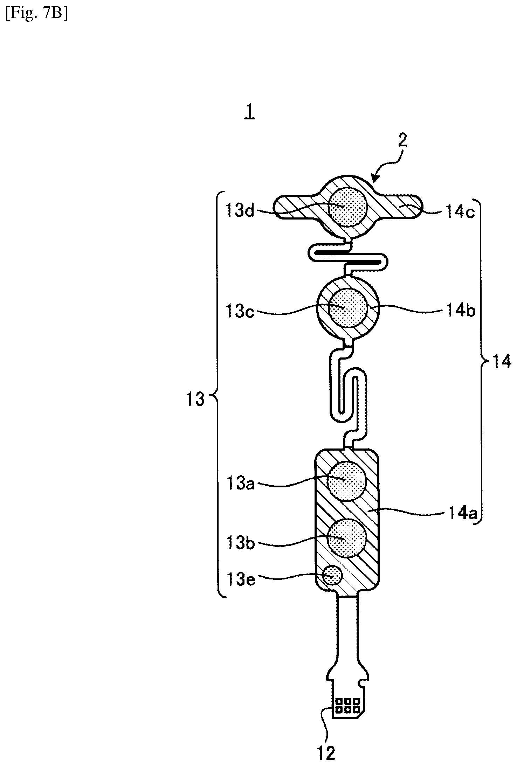

Referring to FIGS. 7A and 7B, next, a modification of the neutral electrode 6 will be described.

In the above-described embodiment, the neutral electrode 6 is disposed proximal to the negative electrode 3a. However, the position where the neutral electrode 6 is disposed can be adequately set in so far as noises flowing through the body electrode 1 can be blocked. Hereinafter, a preferred example of the position where the neutral electrode 6 is disposed will be described.

As illustrated in FIGS. 7A and 7B, the neutral electrode 6 in the modification is disposed at a position that is closer to the connector 12 as compared with the stimulation electrode 3. The distance with respect to the connector 12 is short, and therefore the neutral electrode wiring part 11 can be shortened. Consequently, the production cost can be reduced.

In the case where the neutral electrode 6 is disposed in the upper island portion 2a or the intermediate island portion 2b, a part of the neutral electrode wiring part 11 must be disposed in the first connection portion 2d or the second connection portion 2e, and therefore it is required to thicken the first connection portion 2d or the second connection portion 2e. As a result, the production cost is raised by an amount corresponding to the thickened part of the first connection portion 2d or the second connection portion 2e. As compared with the case where the neutral electrode 6 is disposed in the upper island portion 2a or the intermediate island portion 2b, therefore, the production cost can be reduced.

### Other Examples

Other examples will be described below.

In the body electrode 1 of the above-described embodiment, the stimulation electrode 3, the active electrode 4, and the reference electrode 5 are mounted so that a straight line connects their centers. However, the stimulation electrode 3, the active electrode 4, and the reference electrode 5 may be mounted such that they are deviated in the lateral direction of the body electrode 1.

In the body electrode 1 of the above-described embodiment, the reference electrode 5 is mounted in the upper island portion 2a, and the active electrode 4 is mounted in the intermediate island portion 2b. Alternatively, the active electrode 4 may be mounted on the upper island portion 2a, and the reference electrode 5 may be mounted on the intermediate island portion 2b.

In the body electrode 1 of the above-described embodiment, the negative electrode 3a is disposed at a position that is remoter from the connector 12 with respect to the positive electrode 3b, and the positive electrode 3b is disposed at a position that is closer to the connector 12 with respect to the negative electrode 3a. Alternatively, the position where the positive electrode 3b is mounted and the position where the negative electrode 3a is mounted may be reversed.

Although, in the above-described embodiment, the first direction changing part 2f changes the direction in which the first connection portion 2d extends in the longitudinal direction of the body electrode 1, the direction in which the first connection portion 2d extends may be changed in the lateral direction of the body electrode 1. Likewise, although the second direction changing part 2g changes the direction in which the second connection portion 2e extends in the lateral direction, the direction in which the second connection portion 2e extends may be changed in the longitudinal direction.

Although, in the above-described embodiment, the first direction changing part 2f and the second direction changing part 2g change the directions of the first connection portion 2d and the second connection portion 2e in different directions, respectively, the directions of the first connection portion 2d and the second connection portion 2e may be changed in the same direction.

Although, in the above-described embodiment, a perforation process and a half-cut process have been described as specific examples of the first processed part 101 and the second processed part 102, the presently disclosed subject matter is not limited to this. For example, a spot fixing process in which the both ends of the first processed part 101 and the second processed part 102 are spot-fixed, and the spot-fixed ends are separated from each other may be applied. Alternatively, a cut may be provided at least one side of the first processed part 101 and the second processed part 102.

Although, in the above-described embodiment, the first processed part 101 and the second processed part 102 employ the same process as a mode in which the release sheet 100 can be separated and/or bent, the parts may employ different processes. For example, the first processed part 101 may employ a perforation process, and the second processed part 102 may employ a half-cut process.

Although, in the above-described embodiment, the first processed part 101 and the second processed part 102 enable the release sheet 100 to be separated and/or bent in a horizontal direction, the presently disclosed subject matter is not limited to this. The release sheet 100 may be separated and/or bent in any direction. For example, the first processed part 101 and the second processed part 102 may have a mode in which the release sheet 100 can be separated and/or bent in an oblique direction.

Although, in the above-described embodiment, the first notch 103 and the second notch 104 are disposed in the respective both side ends, the presently disclosed subject matter is not limited to this. The first notch 103 and the second notch 104 may be disposed in only one side, i.e., the right side end or the left side end.

Although, in the above-described embodiment, the body electrode unit that is used to stimulate a nerve leading to a muscle of a subject is stimulated and to monitor the degree of relaxation of the reacting muscle based on a physiological signal of the muscle has been described, the presently disclosed subject matter is not limited to this. For example, the presently disclosed subject matter may be applied also to a body electrode unit that is used in Holter electrocardiography.

Although, in the above-described embodiment, the electrodes and the electric stimulator are connected to each other through the respective wiring parts, the electrodes and the electric stimulator may be connected to each other through a wireless function.

The way the body electrode unit U is used is not limited to the example illustrated in FIGS. 4A to 5D. That is, as described in the following examples, the body electrode unit U may be used in different ways. Thus, it is possible to provide options for a medical person in using the body electrode unit U.

According to a first example, first, the lower sheet portion 100c is separated along the first processed part 101, the lower island portion 2c is peeled off from the lower sheet portion 100c, and the stimulation electrode adhesive tape 14a is attached to the subject. Next, the intermediate sheet portion 100b is separated along the second processed part 102, the intermediate island portion 2b is peeled off from the intermediate sheet portion 100b, and the active electrode adhesive tape 14b is attached to the subject. Then, the upper island portion 2a is peeled off from the upper sheet portion 100a, and the reference electrode adhesive tape 14c is attached to the subject.

According to a second example, first, the upper island portion 2a is peeled off from the upper sheet portion 100a, the release sheet 100 is bent along the second processed part 102, and the reference electrode adhesive tape 14c is attached to the subject. Next, the intermediate island portion 2b is peeled off from the intermediate sheet portion 100b, the release sheet 100 is bent along the first processed part 101, and the active electrode adhesive tape 14b is attached to the subject. Then, the lower island portion 2c is peeled off from the lower sheet portion 100c, and the stimulation electrode adhesive tape 14a is attached to the subject.

According to a third example, first, the upper sheet portion 100a is separated along the second processed part 102, the upper island portion 2a is peeled off from the upper sheet portion 100a, and the reference electrode adhesive tape 14c is attached to the subject. Next, the intermediate sheet portion 100b is separated along the first processed part 101, the intermediate island portion 2b is peeled off from the intermediate sheet portion 100b, and the active electrode adhesive tape 14b is attached to the subject. Then, the lower island portion 2c is peeled off from the lower sheet portion 100c, and the stimulation electrode adhesive tape 14a is attached to the subject.

As described above, the body electrode unit U has the body electrode 1 and the release sheet 100. The body electrode 1 includes the stimulation electrode 3 and the active electrode 4, and the stimulation electrode 3 and the active electrode 4 that are put together to form a single continuous shape. The release sheet includes the first processed part 101 to which the process for facilitating separation of the release sheet 100 is applied, in the first area 100d between the island lower portion 2c where the stimulation electrode 3 is mounted, and the intermediate island portion 2b where the active electrode 4 is mounted. According to the configuration, the release sheet 100 is separated or bent by the first processed part 101, thereby enabling the stimulation electrode 3 and the active electrode 4 to be attached respectively to the body. Therefore, it is possible to provide a body electrode unit having a plurality of electrodes that are put together to form a single continuous shape, with which each electrode can be attached to a desired position on a body.

According to an aspect of the embodiments described above, a body electrode unit includes a body electrode (for example, the body electrode 1) configured to be attached to a body, and a release sheet (for example, the release sheet 100) to which the body electrode is attached. The body electrode includes at least a first electrode (for example, the stimulation electrode 3) and a second electrode (for example, the active electrode 4), the first electrode and the second electrode being put together to form a single continuous shape. The release sheet includes a first processed part (for example, the first processed part 101) provided in a first area (for example, the first area 100d) between a position (for example, the lower sheet portion 100c) at which the first electrode is attached and a position (for example, the intermediate sheet portion 100b) at which the second electrode is attached, and the first processed part being configured to facilitate at least one of a separation and a bending of the release sheet.

According to another aspect of the embodiments described above, the body electrode may have a third electrode (for example, the reference electrode 5). The first electrode, the second electrode, and the third electrode may be put together to form a single continuous shape. The release sheet may include a second processed part (for example, the second processed part 102) provided in a second area (for example, the second area 100e) between the position at which the second electrode is attached and a position (for example, the upper sheet portion 100a) at which the third electrode is attached, and the second processed part being configured to facilitate at least one of the separation and the bending of the release sheet.

According to another aspect of the embodiments described above, the body electrode includes a first electrode mounting portion (for example, the island lower portion 2c) on which the first electrode is mounted, and which has an adhesiveness, a second electrode mounting portion (for example, the intermediate island portion 2b) on which the second electrode is mounted, and which has an adhesiveness, and a first connection portion (for example, the first connection portion 2d) connecting the first electrode mounting portion and the second electrode mounting portion to each other, and that does not have an adhesiveness.

According to another aspect of the embodiments described above, the body electrode further includes a third electrode mounting portion (for example, the upper island portion 2a) on which the third electrode is mounted, and which has an adhesiveness, and a second connection portion (for example, the second connection portion 2e) connecting the second electrode mounting portion and the third electrode mounting portion to each other, and that does not have an adhesiveness.

According to another aspect of the embodiments described above, a notch (for example, the first notch 103) is provided at a side end of the first processed part.

According to another aspect of the embodiments described above, the first processed part is partially cut in a thickness direction of the release sheet, or is perforated.

According to another aspect of the embodiments described above, the first electrode mounting portion includes a neutral electrode (for example, the neutral electrode 6) configured to eliminate a noise flowing through the body electrode.

According to another aspect of the embodiments described above, the first electrode includes a negative electrode (for example, the negative electrode 3a) and a positive electrode (for example, the positive electrode 3b), and the neutral electrode is mounted closer to the negative electrode than to the positive electrode.

While the present invention has been described with reference to certain exemplary embodiments thereof, the scope of the present invention is not limited to the exemplary embodiments described above, and it will be understood by those skilled in the art that various changes and modifications may be made therein without departing from the scope of the present invention as defined by the appended claims.

This application claims priority to Japanese Patent Application No. 2019-183803 filed on October 4, 2019.

## Claims

1. A body electrode unit (U) comprising:
a body electrode (1) configured to be attached to a body; and
a release sheet (100) to which the body electrode is attached,
wherein the body electrode comprises at least a first electrode and a second electrode, the first electrode and the second electrode being put together to form a single continuous shape, **characterized in that** the release sheet comprises a first processed part configured to facilitate at least one of a separation and a bending of the release sheet, the first processed part being provided in a first area between a position at which the first electrode is attached and a position at which the second electrode is attached.

2. The body electrode unit according to claim 1, further comprising a third electrode,
wherein the first electrode, the second electrode, and the third electrode are put together to form the single continuous shape, and
wherein the release sheet further comprises a second processed part configured to facilitate at least one of the separation and the bending of the release sheet, the second processed part being provided in a second area between the position at which the second electrode is attached and a position at which the third electrode is attached.

3. The body electrode unit according to claim 2, wherein the body electrode comprises:
a first electrode mounting portion on which the first electrode is mounted, the first electrode mounting portion being adhesive;
a second electrode mounting portion on which the second electrode is mounted, the second electrode mounting portion being adhesive; and
a first connection portion connecting the first electrode mounting portion and the second electrode mounting portion to each other, the first connection portion being non-adhesive.

4. The body electrode unit according to claim 3, wherein the body electrode further comprises:
a third electrode mounting portion on which the third electrode is mounted, the third electrode mounting portion being adhesive; and
a second connection portion connecting the second electrode mounting portion and the third electrode mounting portion to each other, the second connection portion being non-adhesive.

5. The body electrode unit according to any one of claims 1 to 4, wherein a notch is at a side end of the first processed part.

6. The body electrode unit according to any one of claims 1 to 5, wherein the first processed part is partially cut in a thickness direction of the release sheet.

7. The body electrode unit according to any one of claims 1 to 5, wherein the first processed part is perforated.

8. The body electrode unit according to claim 3, wherein the first electrode mounting portion includes a neutral electrode configured to eliminate a noise flowing through the body electrode.

9. The body electrode unit according to claim 8, wherein the first electrode comprises a negative electrode and a positive electrode, and wherein the neutral electrode is mounted closer to the negative electrode than to the positive electrode.

## Patentansprüche

1. Eine Körperelektrodeneinheit (U), aufweisend:
eine Körperelektrode (1), die so konfiguriert ist, dass sie an einem Körper angebracht werden kann; und
eine Abziehfolie (100), an der die Körperelektrode angebracht ist,
wobei die Körperelektrode mindestens eine erste Elektrode und eine zweite Elektrode aufweist, wobei die erste Elektrode und die zweite Elektrode zusammengefügt sind, um eine einzige kontinuierliche Form zu bilden, **dadurch gekennzeichnet, dass**
die Abziehfolie einen ersten bearbeiteten Teil aufweist, der so konfiguriert ist, dass er das Trennen und/oder Biegen der Abziehfolie erleichtert, wobei der erste bearbeitete Teil in einem ersten Bereich zwischen einer Position, an der die erste Elektrode angebracht ist, und einer Position, an der die zweite Elektrode angebracht ist, vorgesehen ist.

2. Die Körperelektrodeneinheit gemäß Anspruch 1, die ferner eine dritte Elektrode aufweist,
wobei die erste Elektrode, die zweite Elektrode und die dritte Elektrode zusammengefügt sind, um eine einzige kontinuierliche Form zu bilden, und
wobei die Trennfolie ferner ein zweites verarbeitetes Teil aufweist, das so konfiguriert ist, dass es zumindest eines von Trennen und Biegen der Trennfolie erleichtert, wobei das zweite verarbeitete Teil in einem zweiten Bereich zwischen der Position, an der die zweite Elektrode angebracht ist, und einer Position, an der die dritte Elektrode angebracht ist, bereitgestellt wird.

3. Die Körperelektrodeneinheit nach Anspruch 2, wobei die Körperelektrode aufweist:
einen ersten Elektrodenbefestigungsabschnitt, an dem die erste Elektrode befestigt ist, wobei der erste Elektrodenbefestigungsabschnitt klebend ist;
einen zweiten Elektrodenbefestigungsabschnitt, an dem die zweite Elektrode befestigt ist, wobei der zweite Elektrodenbefestigungsabschnitt klebend ist; und
einen ersten Verbindungsabschnitt, der den ersten Elektrodenbefestigungsabschnitt und den zweiten Elektrodenbefestigungsabschnitt miteinander verbindet, wobei der erste Verbindungsabschnitt nicht klebend ist.

4. Die Körperelektrodeneinheit nach Anspruch 3, wobei die Körperelektrode ferner aufweist:
einen dritten Elektrodenbefestigungsabschnitt, an dem die dritte Elektrode befestigt ist, wobei der dritte Elektrodenbefestigungsabschnitt klebend ist; und
einen zweiten Verbindungsabschnitt, der den zweiten Elektrodenbefestigungsabschnitt und den dritten Elektrodenbefestigungsabschnitt miteinander verbindet, wobei der zweite Verbindungsabschnitt nicht klebend ist.

5. Die Körperelektrodeneinheit gemäß einem der Ansprüche 1 bis 4, wobei sich an einem Seitenende des ersten bearbeiteten Teils eine Kerbe befindet.

6. Die Körperelektrodeneinheit gemäß einem der Ansprüche 1 bis 5, wobei das erste bearbeitete Teil teilweise in einer Dickenrichtung der Trennfolie geschnitten ist.

7. Die Körperelektrodeneinheit gemäß einem der Ansprüche 1 bis 5, wobei das erste bearbeitete Teil perforiert ist.

8. Die Körperelektrodeneinheit nach Anspruch 3, wobei der erste Abschnitt zur Elektrodenbefestigung eine neutrale Elektrode umfasst, die so konfiguriert ist, dass sie ein durch die Körperelektrode fließendes Rauschen eliminiert.

9. Die Körperelektrodeneinheit nach Anspruch 8, wobei die erste Elektrode eine negative Elektrode und eine positive Elektrode aufweist und
wobei die neutrale Elektrode näher an der negativen Elektrode als an der positiven Elektrode angebracht ist.

## Revendications

1. Unité électrode corporelle (U) comprenant :
une électrode corporelle (1) configurée pour être fixée à un corps ; et
une feuille de décollement (100) à laquelle l'électrode corporelle est fixée,
dans laquelle l'électrode corporelle comprend au moins une première électrode et une seconde électrode, la première électrode et la seconde électrode étant placées ensemble pour former une forme continue unique,
**caractérisée en ce que** la feuille de décollement comprend une première pièce transformée configurée pour faciliter au moins l'une d'une séparation et d'une flexion de la feuille de décollement, la première pièce transformée étant disposée dans une première zone entre une position à laquelle la première électrode est fixée et une position à laquelle la seconde électrode est fixée.

2. Unité électrode corporelle selon la revendication 1, comprenant en outre une troisième électrode,
dans laquelle la première électrode, la seconde électrode, et la troisième électrode sont placées ensemble pour former la forme continue unique, et
dans laquelle la feuille de décollement comprend en outre une seconde pièce transformée configurée pour faciliter au moins l'une de la séparation et de la flexion de la feuille de décollement, la seconde pièce transformée étant disposée dans une seconde zone entre la position à laquelle la seconde électrode est fixée et une position à laquelle la troisième électrode est fixée.

3. Unité électrode corporelle selon la revendication 2, dans laquelle l'électrode corporelle comprend :
une portion de montage de première électrode sur laquelle la première électrode est montée, la portion de montage de première électrode étant adhésive ;
une portion de montage de seconde électrode sur laquelle la seconde électrode est montée, la portion de montage de seconde électrode étant adhésive ; et
une première portion de connexion connectant la portion de montage de première électrode et la portion de montage de seconde électrode l'une à l'autre, la première portion de connexion étant non adhésive.

4. Unité électrode corporelle selon la revendication 3, dans laquelle l'électrode corporelle comprend en outre :
une portion de montage de troisième électrode sur laquelle la troisième électrode est montée, la portion de montage de troisième électrode étant adhésive ; et
une seconde portion de connexion connectant la portion de montage de seconde électrode et la portion de montage de troisième électrode l'une à l'autre, la seconde portion de connexion étant non adhésive.

5. Unité électrode corporelle selon l'une quelconque des revendications 1 à 4, dans laquelle une encoche se trouve à une extrémité latérale de la première pièce transformée.

6. Unité électrode corporelle selon l'une quelconque des revendications 1 à 5, dans laquelle la première pièce transformée est partiellement coupée dans un sens de l'épaisseur de la feuille de décollement.

7. Unité électrode corporelle selon l'une quelconque des revendications 1 à 5, dans laquelle la première pièce transformée est perforée.

8. Unité électrode corporelle selon la revendication 3, dans laquelle la portion de montage de première électrode inclut une électrode neutre configurée pour éliminer un bruit s'écoulant à travers l'électrode corporelle.

9. Unité électrode corporelle selon la revendication 8, dans laquelle la première électrode comprend une électrode négative et une électrode positive, et
dans laquelle l'électrode neutre est montée plus proche de l'électrode négative que de l'électrode positive.
